# EUROPÄISCHE PATENTSCHRIFT

(11) **EP 1 542 579 B1**
(45) Veröffentlichungstag und Bekanntmachung des Hinweises auf die Patenterteilung: **13.10.2010**
(21) Anmeldenummer: 03747899.7
(22) Anmeldetag: 14.08.2003
(51) Int. Cl.: A61B 1/12, A61M 1/00

(54) **MEDIZINISCHES INSTRUMENT ZUM SAUGEN UND SPÜLEN UND VERFAHREN ZU SEINER HERSTELLUNG**
MEDICAL INSTRUMENT FOR SUCTION AND CLEANING AND RELATED METHOD FOR MAKING SAME
INSTRUMENT MEDICAL D'ASPIRATION ET DE NETTOYAGE ET PROCEDE DE FABRICATION ASSOCIE

(30) Priorität: 20.09.2002 DE 10245009
(43) Veröffentlichungstag der Anmeldung: 22.06.2005
(73) Patentinhaber: Karl Storz GmbH & Co. KG, 78532 Tuttlingen (DE)
(72) Erfinder: SCHMIDBERGER, Jochen, 72355 Schömberg (DE); EFINGER, Andreas, 78604 Rietheim (DE)
(74) Vertreter: Heuckeroth, Volker
(86) Internationale Anmeldenummer: PCT/EP2003/009025
(87) Internationale Veröffentlichungsnummer: WO 2004/032733

(56) Entgegenhaltungen:
- DE-A- 3 629 858
- DE-A- 19 633 124
- FR-A- 2 588 744
- US-A- 5 575 756
- US-B1- 6 234 993

## Beschreibung

Die Erfindung betrifft ein medizinisches Instrument zum Saugen und Spülen, mit einem lang erstreckten Schaft, der einen ersten Kanal, der als Saugkanal dient, und einen zweiten Kanal aufweist, der als Spülkanal dient, wobei der Schaft ein im Querschnitt geschlossenes erstes Rohr aufweist, in dem der eine der Kanäle vorhanden ist, wobei der Schaft ein über seine Länge im Querschnitt offenes zweites Rohr aufweist, das mit seiner offenen Längsseite auf eine Außenseite des ersten Rohrs aufgesetzt und an diesem dichtend befestigt ist, wobei der andere Kanal im Zwischenraum zwischen der Außenseite des ersten Rohrs und einer Innenseite des zweiten Rohrs ausgebildet ist, wobei ferner am proximalen Ende des Schafts ein den Schaft umgebendes Verbindungsteil zum Verbinden des Schafts mit einem Handgriff angeordnet ist.

Die Erfindung betrifft ferner ein Verfahren zum Herstellen eines solchen medizinischen Instruments.

Ein Instrument der eingangs genannten Art ist aus dem Dokument DE 196 33 124 A bekannt.

Ein solches Instrument wird im Rahmen der Chirurgie bei Eingriffen am menschlichen oder tierischen Körper dazu verwendet, das Operationsgebiet mit frischer Spülflüssigkeit zu spülen und die Spülflüssigkeit sowie Blut und Gewebereste wieder aus dem Operationsgebiet abzusaugen. Bei endoskopischen Eingriffen dient das Spülen außerdem der Aufrechterhaltung der freien Sicht durch ein Endoskop.

Um gleichzeitig saugen und spülen zu können, ist es erforderlich, daß ein solches Instrument einen Schaft aufweist, der zwei Kanäle besitzt, und zwar einen Saugkanal und einen Spülkanal. Da die frische Spülflüssigkeit sich mit der abgesaugten Flüssigkeit nicht vermischen sollte, bevor sie in den Operationsraum gelangt, ist es weiterhin erforderlich, daß der Spülkanal von dem Saugkanal so separiert ist, daß sich Spülflüssigkeit und abgesaugte Flüssigkeit nicht miteinander im Schaft vermischen.

Aus dem DE-Firmenprospekt der Firma Karl Storz GmbH & Co. KG, "STORZ Karl Storz-Endoskope", Band "Laparoskopie", 3. Ausgabe 2/99, Seite IS-ACC 5 A ist ferner ein Instrument bekannt, dessen Schaft für den Spülkanal ein erstes Rohr und für den Saugkanal ein zweites Rohr aufweist, wobei das den Spülkanal bildende erste Rohr in das den Saugkanal bildende zweite Rohr als Einsatz eingeschoben ist. Der Saugkanal wird entsprechend zwischen der Außenseite des den Spülkanal bildenden ersten Rohrs und der Innenseite des zweiten Rohrs gebildet. Der Schaft dieses Instruments ist somit aus zwei im wesentlichen nicht miteinander verbundenen Rohren zusammengesetzt, woraus verschiedene Nachteile resultieren können. Zum einen besitzen einzelne Rohre eine geringere Biegestabilität, und zum anderen müssen sie jeweils einzeln nach außen mit entsprechenden Dichtungsmaßnahmen abgedichtet werden, beispielsweise durch O-Ringe, die einem Verschleiß unterliegen. Die ineinandergeschobene Anordnung aus Spülrohr und Saugrohr hat darüber hinaus den Nachteil, daß in dem Saugrohr bzw. in dem dünnen Spülrohr kein Raum zur Einführung eines Endoskops verbleibt, wenn nicht der Querschnitt des Gesamtschaftes über Maßen groß gewählt wird, was jedoch im Rahmen der minimal-invasiven Chirurgie, die durch kleinste Inzisionen am Körper erfolgt, unerwünscht ist. Darüber hinaus ist vor der Anwendung des Instruments eine Montage und vor der Reinigung des Instruments eine Demontage der einzelnen Rohre erforderlich, was die Handhabung des Instruments erschwert.

Aus dem Dokument DE 36 29 858 A ist ein medizinisches Instrument in Form eines Endoskops bekannt, dessen Handhabungsteil mit dem Schaft des Endoskops unter Verwendung von Klebstoff verbunden ist. Dieses Instrument weist einen Spülkanal und einen Instrumenteneinführkanal auf. Der Spülkanal ist als Innenrohr in dem Schaft ausgebildet.

Ferner ist aus der WO 00/48505 ein Endoskop bekannt, das einen Außenschaft aufweist, in dem ein gekammerter Profilschaft angeordnet ist, der eine Kammer aufweist, die als Spülkanal dient, und eine davon separierte weitere Kammer, die als Saugkanal dient. In dem Profilschaft sind des weiteren ein Optikkanal zur Aufnahme eines optischen Systems sowie weitere Kammern zur Aufnahme eines lichtzuführenden Systems, beispielsweise von Lichtleitfasern, vorhanden. Bei dieser Ausgestaltung sind zwar der Spülkanal und der Saugkanal einteilig miteinander verbunden, jedoch ist ein solcher Profilschaft in seiner Herstellung relativ aufwendig, da er sich nur in einem Strangpreßverfahren herstellen läßt.

Der Erfindung liegt die Aufgabe zugrunde, ein medizinisches Instrument der eingangs genannten Art dahingehend zu verbessern, daß es technisch einfach und kostengünstig herstellbar ist, und daß zugleich eine Abdichtung des Saug- gegen den Spülkanal erreicht wird.

Der Erfindung liegt ferner die Aufgabe zugrunde, ein Verfahren zur Herstellung eines solchen Instruments anzugeben.

Erfindungsgemäß wird die zuerst genannte Aufgabe hinsichtlich des eingangs genannten medizinischen Instruments dadurch gelöst, daß der Schaft in das Verbindungsteil mittels eines aushärtenden Klebstoffes eingeklebt ist, und daß der Klebstoff das erste Rohr und das zweite Rohr in deren proximalem Endbereich gegeneinander abdichtet, wobei ein proximales offenes Ende des zweiten Rohrs mittels desselben Klebstoffs oder eines weiteren Klebstoffs verschlossen ist.

Bei dem erfindungsgemäßen Instrument wird der Schaft nicht durch zwei einzelne Rohre gebildet, die ineinander eingesetzt und wieder voneinander getrennt werden können, sondern der Schaft des erfindungsgemäßen Instruments weist zur Ausbildung des einen der beiden Kanäle ein über seine Länge im Querschnitt offenes zweites Rohr auf, das von seiner offenen Längsseite auf eine Außenseite des ersten Rohrs aufgesetzt und an diesem dichtend befestigt ist. Auf diese Weise überspannt das zweite Rohr nur einen Teilumfang des ersten Rohres, wodurch der Gesamtquerschnitt des Schaftes zum einen gering gehalten wird, und zum anderen die Möglichkeit besteht, in das erste Rohr zusätzlich ein Endoskop einschieben zu können, da der Innenraum des ersten Rohres nunmehr frei bleibt. Das auf das erste Rohr aufgesetzte und mit diesem fest verbundene zweite Rohr erhöht darüber hinaus die Biegesteifigkeit der Gesamtanordnung aus Saug- und Spülrohr. Ein weiterer wesentlicher Vorteil des erfindungsgemäßen Instruments besteht darin, daß der Zugang zu dem ersten Rohr und der Zugang zu dem zweiten Rohr zum Einleiten bzw. Abführen von Flüssigkeit nicht durch das jeweils andere Rohr hindurch erfolgen muß, weil das zweite Rohr nur einen Teilumfang der Außenseite des ersten Rohres überdeckt, während der übrige Umfang des ersten Rohres frei bleibt. Auch die Herstellung des Schafts des erfindungsgemäßen Instruments ist wesentlich vereinfacht, da sich das zweite Rohr mit einfachen Fügeverfahren, wie beispielsweise Kleben, Löten oder Schweißen, leicht auf der Außenseite des ersten Rohres dauerhaft befestigen läßt.

Am proximalen Ende des Schafts ist ein den Schaft umgebendes Verbindungsteil zum Verbinden des Schafts mit einem Handgriff angeordnet, wobei der Schaft in das Verbindungsteil mittels eines aushärtenden Klebstoffs eingeklebt ist.

Hierbei ist von Vorteil, daß nicht nur der Schaft insgesamt einteilig ausgebildet ist, sondern auch die Anordnung aus dem Schaft und dem Verbindungsteil zum Verbinden des Schafts mit einem Handgriff ebenfalls einteilig ausgebildet ist, wodurch der Aufwand bei der Montage und bei der Demontage des Instruments weiter vereinfacht wird. Das Verbinden des Schafts mit dem Verbindungsteil mittels eines aushärtenden Klebstoffes hat den Vorteil, daß es herstellungstechnisch einfach und kostengünstig durchführbar ist, und daß darüber hinaus der Klebstoff etwaige Zwischenräume zwischen dem Verbindungsteil und dem Schaft ausfüllen und so das Verbindungsteil gegen die Außenseite des Schafts ohne weitere Maßnahmen ausreichend abdichten kann, so daß zwischen den Schaft und das Verbindungsteil keine Verunreinigungen oder dergleichen eindringen können.

Dabei dichtet der Klebstoff das erste Rohr und das zweite Rohr in deren proximalen Endbereich gegeneinander ab.

Hierbei ist weiterhin von Vorteil, daß auf Dichtungsmaßnahmen wie das Vorsehen von O-Ringen vollständig verzichtet werden kann, um die beiden Rohre so gegeneinander abzudichten, daß sich die Spülflüssigkeit nicht mit der abgesaugten Flüssigkeit vermischen kann.

In einer bevorzugten Ausgestaltung ist der Zwischenraum zwischen der Außenseite des ersten Rohres und der Innenseite des zweiten Rohres sichelförmig mit geringer Scheitelhöhe ausgebildet.

Hierbei ist von Vorteil, daß der Gesamtquerschnitt des Schafts durch das zweite Rohr über den Querschnitt des ersten Rohrs hinaus nur geringfügig vergrößert wird. Der im Querschnitt sichelförmige Zwischenraum zwischen der Außenseite des ersten Rohrs und der Innenseite des zweiten Rohrs eignet sich besonders für den Spülkanal, da in der Spülflüssigkeit keine festen Stoffe enthalten sind, die sich in den Ecken des sichelförmigen Querschnitts festsetzen können.

In einer weiteren bevorzugten Ausgestaltung weist das erste Rohr weiterhin einen dritten Kanal zur Aufnahme eines Optikschafts eines Endoskops auf, und das zweite Rohr ist auf derjenigen Außenseite des ersten Rohrs aufgesetzt, die von dem verbleibenden freien Querschnitt in dem ersten Rohr, der den ersten oder zweiten Kanal bildet, abgewandt ist.

Diese Anordnung des zweiten Rohres hat den Vorteil, daß die Spülflüssigkeit beim Austreten aus dem distalen Ende des einen Rohrs aufgrund der am distalen Ende des anderen Rohrs anliegenden Saugdruckes am distalen Ende des Optikschafts vorbeigeleitet wird, wodurch sichergestellt werden kann, daß die Sicht durch das Endoskop durch Blut oder Gewebereste nicht beeinträchtigt wird.

In einer weiteren bevorzugten Ausgestaltung überragt das zweite Rohr in Längsrichtung geringfügig die Außenseite des ersten Rohrs, an der es aufgesetzt ist.

Durch diese Maßnahme kann die zuvor beschriebene Wirkung des Vorbeileitens der Spülflüssigkeit an dem lichteintrittsseitigen Ende des Endoskops noch verbessert werden.

In einer weiteren bevorzugten Ausgestaltung ist ein distales Ende des zweiten Rohrs zum ersten Rohr hin umgebördelt, und/oder ein distales Ende des ersten Rohrs ist auf der dem zweiten Rohr abgewandten Außenseite zum zweiten Rohr hin umgebördelt.

Auch diese Maßnahmen tragen vorteilhafterweise zu einer verbesserten Umspülung des distalen Endes des Optikschafts des ggf. in das erste Rohr eingesetzten Endoskops bei, und auch die Saugwirkung des Instruments wird dadurch verbessert.

In einer weiteren bevorzugten Ausgestaltung ist der Saugkanal im ersten Rohr und der Spülkanal im Zwischenraum zwischen der Außenseite des ersten Rohrs und der Innenseite des zweiten Rohrs angeordnet.

Diese Maßnahme ist insbesondere dann, wenn, wie in einer zuvor beschriebenen Ausgestaltung der Zwischenraum zwischen der Innenseite des zweiten Rohrs und der Außenseite des ersten Rohrs sichelförmig mit geringer Scheitelhöhe ist, besonders vorteilhaft, da für die Spülflüssigkeit ein geringerer Querschnitt erforderlich ist als für die abgesaugte Flüssigkeit, die auch feste Stoffe, insbesondere Gewebereste, enthalten kann, so daß der Querschnitt des Schafts mit kombiniertem Saug- und Spülkanal klein gehalten werden kann.

In einer weiteren bevorzugten Ausgestaltung ist das zweite Rohr entlang seiner Längsränder mit der Außenseite des ersten Rohrs durch Kleben, Löten oder Schweißen fest verbunden.

Wenn das zweite Rohr unmittelbar mit seinen Längsrändern mit dem ersten Rohr fest verbunden ist, hat dies den Vorteil, daß sich auf der Außenseite des ersten Rohrs im Bereich der Verbindung mit dem zweiten Rohr keine Ecken oder Nischen bilden, in denen sich Verunreinigungen ansammeln können. Auf diese Weise ist der Schaft des erfindungsgemäßen Instruments leicht zu reinigen. Etwaige Kanten werden dabei durch den Klebstoff, das Lot bzw. das Schweißmaterial ausgefüllt bzw. geglättet.

Besonders bevorzugt ist es dabei, wenn das zweite Rohr entlang seiner Längsränder mit der Außenseite des ersten Rohrs durch Laserschweißen verbunden ist.

Die Verbindung des zweiten Rohrs mit dem ersten Rohr durch Laserschweißen hat den Vorteil, daß mittels Laserschweißen eine sehr exakte und dünne Schweißnaht gebildet werden kann, die einen ausreichenden Schutz gegen Korrosion und Verschmutzungen an der Verbindungsstelle zwischen dem ersten und dem zweiten Rohr bietet.

Hinsichtlich des eingangs genannten Verfahrens zum Herstellen eines medizinischen Instruments zum Saugen und Spülen, das einen lang erstreckten Schaft aufweist, der einen ersten Kanal, der als Saugkanal dient, und einen zweiten Kanal aufweist, der als Spülkanal dient, wobei der Schaft ein erstes Rohr aufweist, in dem der eine der Kanäle angeordnet ist, wobei ein über seine Länge im Querschnitt offenes zweites Rohr mit seiner offenen Längsseite auf eine Außenseite des ersten Rohrs aufgesetzt und an diesem dichtend befestigt wird, derart, dass zwischen der Außenseite des ersten Rohrs und einer Innenseite des zweiten Rohrs ein in Längsrichtung durchgehender Zwischenraum verbleibt, wird die oben an zweiter Stelle genannte Aufgabe dadurch gelöst, daß die miteinander verbundene Einheit aus erstem Rohr und zweitem Rohr anschließend mit einem Verbindungsteil zum Verbinden des Schafts mit einem Handgriff verbunden wird, wobei in dem Verbindungsteil eine Längsbohrung vorhanden ist, oder in das Verbindungsteil vor dem Verbinden mit dem Schaft eine Längsbohrung eingebracht wird, in die der Schaft eingeführt und anschließend mittels eines Klebestoffes eingeklebt wird, so daß der Klebstoff das erste Rohr und das zweite Rohr in deren proximalen Endbereich gegeneinander abdichtet, wobei ein proximales offenes Ende des zweiten Rohrs mittels desselben Klebstoffes oder eines weiteren Klebstoffes verschlossen wird.

Das erfindungsgemäße Herstellungsverfahren hat den Vorteil, daß sich das Instrument mit einem kombinierten Saug- und Spülkanal auf besonders einfache Weise schaffen läßt, indem lediglich das zweite, an einer Längsseite offene Rohr mit seiner offenen Längsseite auf eine Außenseite des ersten Rohrs aufgesetzt und an diesem dichtend befestigt werden muß.

Die miteinander verbundene Einheit aus erstem Rohr und zweitem Rohr wird mit einem Verbindungsteil zum Verbinden des Schafts mit einem Handgriff verbunden.

In dem Verbindungsteil ist eine Längsbohrung vorhanden oder in das Verbindungsteil wird vor dem Verbinden mit dem Schaft eine Längsbohrung eingebracht, in die der Schaft eingeführt und anschließend mittels eines Klebstoffes eingeklebt wird.

Es ist bevorzugt, wenn das zweite Rohr mit dem ersten Rohr durch Kleben, Löten oder Schweißen fest verbunden wird.

Diese Verbindungstechniken sind allesamt leicht und kostengünstig durchführbar und ermöglichen eine dauerhafte mechanisch stabile und dichte Befestigung des zweiten Rohrs an dem ersten Rohr.

Besonders bevorzugt ist es, wenn das zweite Rohr mit dem ersten Rohr durch Laserschweißen verbunden wird.

Wie bereits oben erwähnt, hat Laserschweißen den Vorteil, daß die Schweißnaht ohne starken Materialauftrag sehr dünn und exakt hergestellt werden kann.

In einer weiteren bevorzugten Ausgestaltung wird vor dem Verbinden des zweiten Rohrs mit dem ersten Rohr in das zweite Rohr eine seitliche Bohrung eingebracht.

Diese Maßnahme ist insbesondere dann vorteilhaft, wenn der Zwischenraum zwischen dem ersten Rohr und dem zweiten Rohr nur eine geringe Höhe aufweist, weil dann ein nachträgliches Einbringen der seitlichen Bohrung zu einer Beschädigung des ersten Rohrs führen könnte. Durch das vorherige Einbringen der seitlichen Bohrung wird eine solche Beschädigung des ersten Rohrs sicher vermieden.

Es ist weiterhin bevorzugt, wenn als Klebstoff zum Einkleben des Schafts in das Verbindungsteil ein sich unter Wärmeeinwirkung zunächst verflüssigender und anschließend aushärtender Klebstoff verwendet wird.

In einer weiteren bevorzugten Ausgestaltung wird die seitliche Bohrung in dem zweiten Rohr vor dem Einfügen des Klebstoffes mittels eines anschließend wieder abnehmbaren Stopfens verschlossen.

Hierbei wird der Vorteil erzielt, daß ein Eindringen des Klebstoffes nach seiner Verflüssigung in den Zwischenraum zwischen der Außenseite des ersten Rohrs und der Innenseite des zweiten Rohrs vermieden wird.

Dabei wird vorzugsweise als Stopfen ein rasch aushärtender wieder abziehbarer weiterer Klebstoff verwendet.

Die Verwendung eines solchen Klebstoffs hat den Vorteil, daß er sich nach dem Aushärten wieder leicht entfernen läßt und daß die seitliche Bohrung in dem zweiten Rohr besonders rasch verschlossen werden kann, wodurch das Herstellungsverfahren zeitgünstig durchführbar ist.

In einer weiteren bevorzugten Ausgestaltung wird ein proximales offenes Ende des zweiten Rohrs vor dem Einbringen des Klebstoffes verschlossen.

Hierbei ist von Vorteil, daß auch ein Eindringen des Klebstoffes zum Verbinden des Schaftes mit dem Verbindungsteil in das offene proximale Ende des zweiten Rohrs vermieden wird. Die Verwendung eines rasch aushärtenden Klebstoffs, der jedoch im Unterschied zu dem zuvor genannten abziehbaren Klebstoff nicht wieder abziehbar ist, hat den Vorteil, daß das proximale offene Ende des Zwischenraums wischen der Innenseite des zweiten Rohrs und der Außenseite des ersten Rohrs mit sehr geringem Zeitaufwand verschlossen werden kann.

In einer weiteren bevorzugten Ausgestaltung wird nach dem Einkleben des Schafts in das Verbindungsteil in das erste Rohr an einer Stelle, die nicht von dem zweiten Rohr bedeckt ist, eine seitliche Bohrung durch den ausgehärteten Klebstoff hindurch eingebracht.

Hierbei ist von Vorteil, daß der Zugang zu dem ersten Rohr, der durch die seitliche Bohrung gebildet wird, bereits abgedichtet ist, ohne daß weitere Dichtungsmaßnahmen erforderlich werden, weil die seitliche Bohrung durch den ausgehärteten Klebstoff hindurch erfolgt.

Weitere Vorteile und Merkmale ergeben sich aus der nachfolgenden Beschreibung und der beigefügten Zeichnung.

Es versteht sich, daß die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Fig. 1: ein medizinisches Instrument zum Saugen und Spülen in Seitenansicht in zwei Teilbildern;
- Fig. 2: einen Längsschnitt durch das untere Teilbild des In- struments in Fig. 1;
- Fig. 3: einen Längsschnitt durch das obere Teilbild des In- struments in Fig. 1;
- Fig. 4: einen Schnitt entlang der Linie IV-IV in Fig. 3;
- Fig. 5: ein Einzelteil des Instruments in Fig. 1 in Allein- stellung im Längsschnitt; und
- Fig. 6: eine perspektivische Gesamtdarstellung des Instru- ments in Fig. 1 mit einem Handgriff in gegenüber Fig. 1 verkleinertem Maßstab, wobei der Handgriff in einer Stellung kurz vor dem Verrasten mit dem Verbindungs- teil des Schafts gezeigt ist.

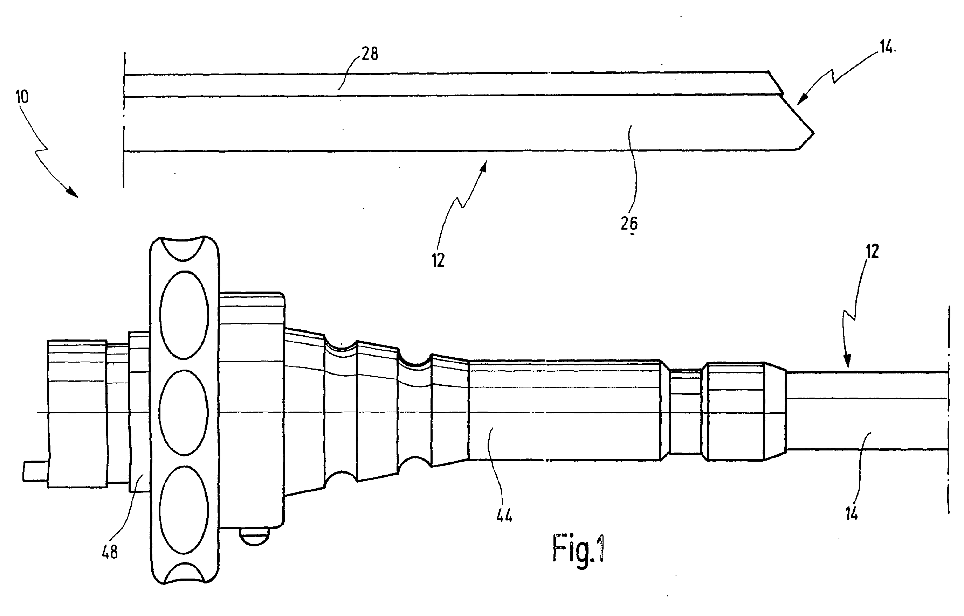

In Fig. 1 ist ein mit dem allgemeinen Bezugszeichen 10 versehenes medizinisches Instrument zum Saugen und Spülen in zwei Teilbildern dargestellt. Das obere Teilbild in Fig. 1 zeigt einen distalen Abschnitt des Instruments 10, und das untere Teilbild einen sich an den distalen Abschnitt anschließenden proximalen Abschnitt des Instruments 10.

Das Instrument 10 wird bei chirurgischen Eingriffen, insbesondere im Rahmen der minimal-invasiven Chirurgie, zum Zuführen von Spülflüssigkeit in ein Operationsgebiet und zum Absaugen von Flüssigkeiten und Geweberesten aus dem Operationsgebiet verwendet. Darüber hinaus ermöglicht das Instrument 10 eine visuelle Kontrolle durch ein Endoskop, wie hiernach noch beschrieben wird.

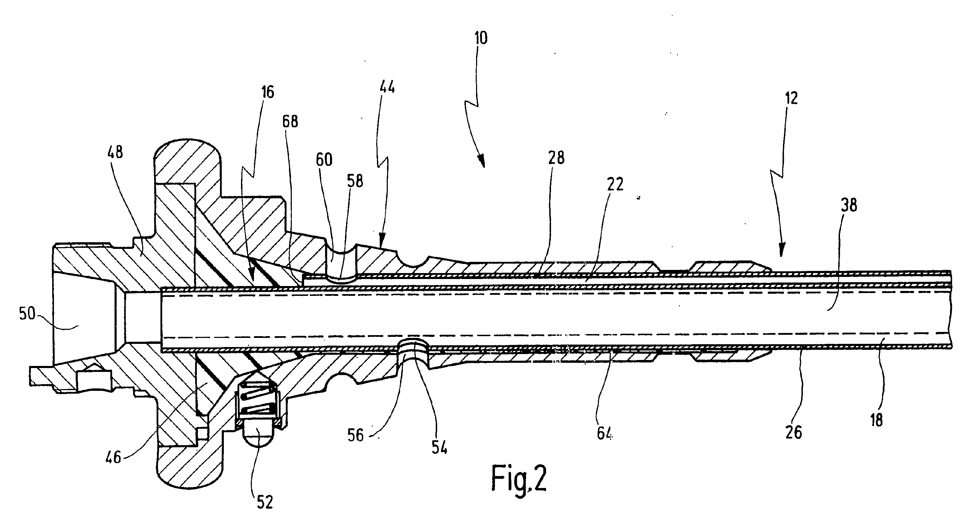

Das Instrument 10 weist einen lang erstreckten Schaft 12 auf, der ein distales Ende 14 und ein proximales Ende 16 (vgl. Fig. 2) aufweist.

Der Schaft 12 weist einen ersten Kanal 18 auf, der als Saugkanal dient und der am distalen Ende 14 des Schafts 12 entsprechend eine Saugöffnung 20 aufweist.

Der Schaft 12 weist weiterhin einen zweiten Kanal 22 auf, der von dem ersten Kanal 18 getrennt ist und der am distalen Ende 14 des Schafts 12 entsprechend eine Spülöffnung 24 aufweist.

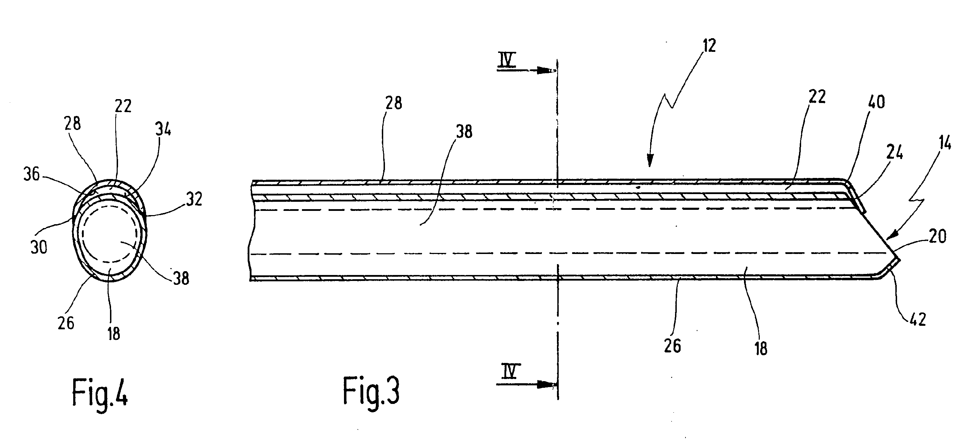

Der Schaft 12 weist ein im Querschnitt geschlossenes (vgl. Fig. 4) erstes Rohr 26 auf, in dem der erste Kanal 18 (Saugkanal) angeordnet ist. Der Schaft 12 weist weiterhin ein über seine Länge im Querschnitt offenes (vgl. Fig. 4) zweites Rohr 28 auf, das mit seiner offenen Längsseite, hier mit seinen Längsrändern 30 und 32 (vgl. Fig. 4), auf eine umfänglich begrenzte Außenseite 34 des ersten Rohrs 26 aufgesetzt und an diesem dichtend befestigt ist. Der den Spülkanal bildende zweite Kanal 22 in dem Schaft 12 ist dabei im Zwischenraum zwischen der Außenseite 34 des ersten Rohrs 26, auf der das zweite Rohr 28 aufgesetzt ist, und einer Innenseite 36 des zweiten Rohrs 28 ausgebildet.

Das zweite Rohr 28 ist entlang seiner Längsränder 30 und 32 mit dem ersten Rohr 26 durch eine durchgehende Laserschweißnaht dicht verbunden.

Der Querschnitt des ersten Rohrs 26 ist gemäß Fig. 4 nicht exakt rund, sondern weist in seinem dem zweiten Rohr 28 abgewandten Bereich eine ovale Ausbuchtung auf. Das zweite Rohr 28 ist im wesentlichen aus einem Vollrohr, hier Zylinderrohr gebildet, das etwa hälftig aufgeschnitten ist. Der den zweiten Kanal 22 bildende Zwischenraum zwischen der Außenseite 34 des ersten Rohrs 26 und der Innenseite 36 des zweiten Rohrs 28 ist sichelförmig mit geringer Scheitelhöhe ausgebildet, so daß der Gesamtquerschnitt des Schafts 12 nur geringfügig größer als der Querschnitt des ersten Rohrs 26 alleine ist. Der gesamte Querschnitt des Schafts 12 ist gemäß Fig. 4 etwa oval geformt.

Das erste Rohr 26 weist weiterhin einen in Fig. 2 und 4 mit unterbrochenen Linien angedeuteten dritten Kanal 38 auf, in den ein nicht dargestellter Optikschaft eines Endoskops eingeführt werden kann, der dann bis zum distalen Ende 14 des Schafts 12 reicht. Der weitere Kanal 38 ist von dem ersten Kanal 18, der den Saugkanal bildet, nicht durch eine Zwischenwand getrennt. Der Saugkanal 18 wird bei in den Kanal 38 eingesetztem Optikschaft durch den verbleibenden Zwischenraum zwischen dem Optikschaft und dem ersten Rohr 26 gebildet, wie aus Fig. 2 bis 4 hervorgeht.

Das zweite Rohr 28 ist dabei auf derjenigen Außenseite 34 des ersten Rohrs 26 aufgesetzt, die von dem den Saugkanal bildenden ersten Kanal 18 im ersten Rohr 26 abgewandt ist. Der erste Kanal 18 und der zweite Kanal 22 liegen sich somit bezüglich des weiteren Kanals 38 diametral gegenüber.

Wie insbesondere aus Fig. 3 hervorgeht, überragt das zweite Rohr 28 geringfügig die Außenseite 34 des ersten Rohrs 26, an der es aufgesetzt ist. Ein distales Ende 40 des zweiten Rohrs 28, an dem die Spülöffnung 24 ausgebildet ist, ist zum ersten Rohr 26 hin umgebördelt. Ebenso ist ein distales Ende 42 des ersten Rohrs 26 auf der dem zweiten Rohr 28 abgewandten Außenseite zum zweiten Rohr 28 hin umgebördelt.

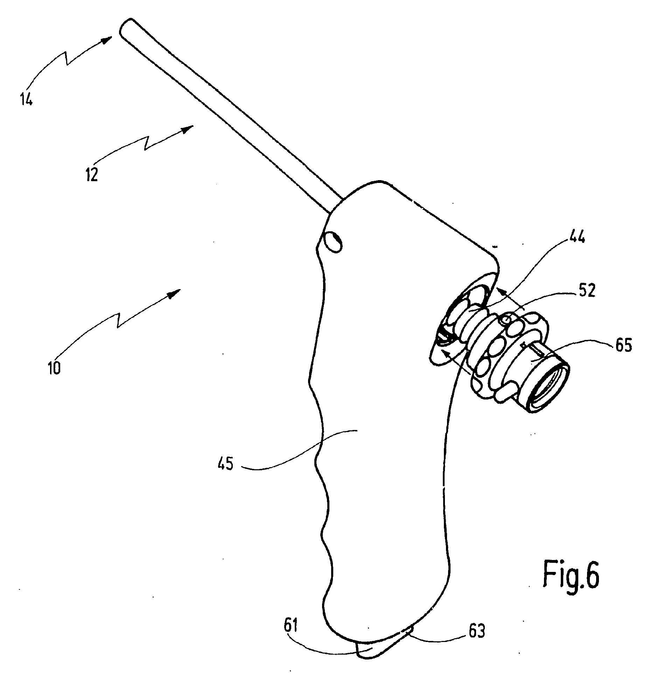

Am proximalen Ende 16 des Schafts 12 ist ein den Schaft 12 umgebendes Verbindungsteil 44 zum Verbinden des Schafts 12 mit einem in Fig. 6 dargestellten Handgriff 45 angeordnet, das mit dem Schaft 12 fest verbunden ist. Die feste Verbindung des Schafts 12 mit dem Verbindungsteil 44 ist mittels eines aushärtenden Klebstoffes 46 realisiert, der im wesentlichen alle Zwischenräume zwischen der Innenseite des Verbindungsteils 44 und der Außenseite des Schafts 12 ausfüllt, ohne dabei in das Innere des Schafts 12, d.h. weder in den Kanal 22 noch in den Kanal 18 bzw. den Kanal 38 einzudringen. Der Klebstoff 46 dichtet dabei den ersten Kanal 18 gegen den zweiten Kanal 22 an deren proximalem Ende ab. Das Verbindungsteil 44 ist an seinem proximalen Ende mit einem Deckel 48 verschlossen, in dem eine Bohrung 50 zum Einführen des Endoskops in den Kanal 38 vorhanden ist. Diese Bohrung 50 ist bei eingeführtem Endoskop gegen das Endoskop abgedichtet. Gemäß Fig. 6 wird in diese Bohrung 50 das Endoskop eingeführt und mittels einer sogenannten Zehngangkupplung 65 befestigt. An dem Verbindungsteil 44 ist ferner ein Rastknopf 52 vorgesehen, an dem der Handgriff 45 an dem Verbindungsteil 44 festlegbar ist.

Im Bereich des Verbindungsteils 44 ist sowohl für den den Saugkanal bildenden ersten' Kanal 18 als auch für den den Spülkanal bildenden zweiten Kanal 22 jeweils ein Zu- bzw. Abgang vorgesehen. Dazu ist in dem ersten Rohr 26 auf seiner dem zweiten Rohr 28 abgewandten Seite eine seitliche, hier radiale Bohrung 54 vorhanden, die mit einer Bohrung 56 in dem Verbindungsteil 44 fluchtet. Entsprechend ist in dem zweiten Rohr 28 eine Bohrung 58 vorhanden, die mit einer Bohrung 60 in dem Verbindungsteil 44 fluchtet. Auch die Bohrungen 58 und 60 sind hier radial ausgebildet. An dem Handgriff 45 (Fig. 6) sind entsprechende Anschlüsse 61, 63 zum Anschließen eines Saug- und eines Spülschlauches vorhanden, wobei sich von diesen Anschlüssen 61, 63 durch den Handgriff 45 nicht näher dargestellte Kanäle erstrecken, die bei auf dem Verbindungsteil 44 aufgesetzten Handgriff 45 über nicht dargestellte Ventile in die Bohrungen 60 und 56 und damit in die Bohrungen 58 und 54 münden. Der Klebstoff 46 dichtet die Ränder der Bohrungen 54 bis 60 ab.

Mit dem Instrument 10 kann permanent durch den Kanal 18 gesaugt werden, während über den Kanal 22 zu jedem Zeitpunkt die Spülung über ein am nicht dargestellten Handgriff vorhandenes Ventil zugeschaltet werden kann, ohne daß die Saugung über den Kanal 18 dazu unterbrochen werden muß.

Nachfolgend wird nun ein Verfahren zum Herstellen des Instruments 10 näher beschrieben.

In einem ersten Schritt werden das erste Rohr 26 und das zweite Rohr 28 bereitgestellt. Das zweite Rohr 28 kann dabei aus einem Vollrohr, hier Zylinderrohr, durch Aufschneiden entlang einer Halbebene hergestellt werden, so daß das zweite Rohr 28 eine offene Längsseite aufweist. Mit dieser offenen Längsseite wird das zweite Rohr 28 dann mit den Längsrändern 30 und 32 auf die Außenseite 34 des ersten Rohrs 26 aufgesetzt und entlang der Längsränder 30 und 32 mit dem ersten Rohr 26 durch Laserschweißen fest verbunden. Auf diese Weise ist dann der einteilige Schaft 12 fertiggestellt.

Vor dem Befestigen des zweiten Rohrs 28 auf dem ersten Rohr 26 wird noch die seitliche Bohrung 58 in das zweite Rohr 28 eingebracht.

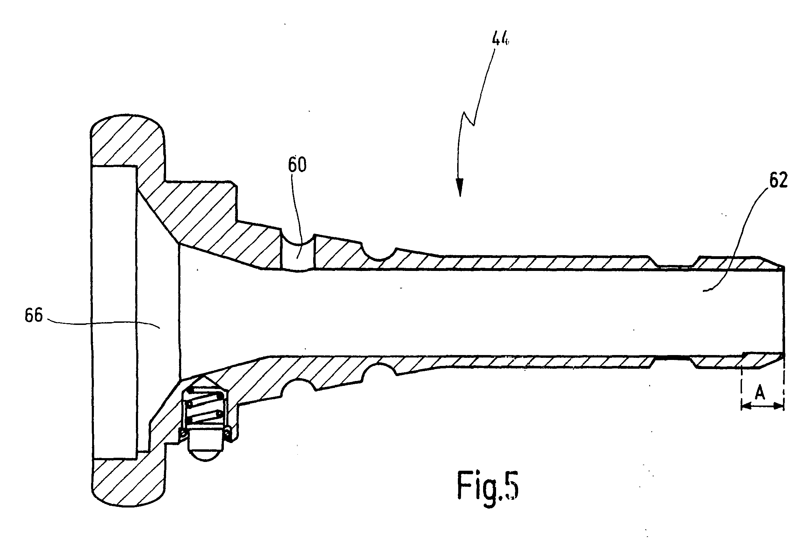

Die miteinander verbundene Einheit aus erstem Rohr 26 und zweitem Rohr 28 wird anschließend mit dem Verbindungsteil 44 verbunden. Das Verbindungsteil 44 wird dabei in der in Fig. 5 dargestellten Form bereitgestellt. Das Verbindungsteil 44 ist ein einstückiges Drehteil, in das zuvor die seitliche Bohrung 60 und eine Längsbohrung 62 eingebracht wurde.

Nachdem der Schaft 12 in das Verbindungsteil 44 in die in Fig. 2 dargestellte Position eingeschoben wurde, in der die Bohrung 58 in dem zweiten Rohr 28 mit der Bohrung 60 in dem Verbindungsteil 44 fluchtet, wird nun mit dem Einfüllen des aushärtenden Klebstoffs 46 begonnen. Der Klebstoff 46 wird über das offene Ende 66 des Verbindungsteils 44 gemäß Fig. 5 eingefüllt, und zwar in den Zwischenraum zwischen dem proximalen Ende des Schafts 16 und dem verbleibenden Raum der Längsbohrung 62 in dem Verbindungsteil 44, ohne daß Klebstoff in das erste Rohr 26 gelangt. Der Klebstoff wird erwärmt und verflüssigt sich dabei, so daß der Klebstoff aufgrund von Kapillarwirkung in den Spalt 64 fließen kann und somit den Schaft 12 gegen das Verbindungsteil 44 abdichtet.

Um zu verhindern, daß dabei Klebstoff in das proximale offene Ende 68 des zweiten Kanals 22 eindringt, wird dieses offene Ende 68 vor dem Einfüllen des Klebstoffs 46 mittels eines rasch aushärtenden weiteren Klebstoffs verschlossen, der in kurzer Zeit aushärtet. Dieser Klebstoff verbleibt dann nach dem Einfüllen des Klebstoffs 46 am proximalen offenen Ende 68 des zweiten Rohrs 28.

Um des weiteren zu verhindern, daß der Klebstoff 46 über die Bohrung 58 in den zweiten Kanal 22 eindringt, wird vor dem Eingießen des Klebstoffs 46 in die Bohrung 58 ein Stopfen mit einem weiteren Klebstoff, der ebenfalls in kurzer Zeit aushärtet, eingesetzt. Hierbei wird ein Klebstoff verwendet, der sich wieder abziehen läßt, um die Bohrung 58 später als Zugang zu dem Spülkanal 22 freizugeben.

Anstatt das proximale offene Ende 68 und die Bohrung 58 vor dem Eingießen des Klebstoffs 46 mittels eines weiteren Klebstoffs zu verschließen, kann auch in Erwägung gezogen werden, den Klebstoff 46 in mehreren Schritten in das Verbindungsteil 44 einzugießen, wobei dann der Effekt ausgenutzt werden kann, daß der Klebstoff zuerst in kleine Spalte fließt, d.h. sich um den Rand der Bohrung 58 plaziert und dann den Rand der Bohrung 58 gegen das Verbindungsteil 44 im ersten Schritt bereits abdichtet, so daß beim schrittweisen Nachgießen des Klebstoffs 46 kein Klebstoff in die Bohrung 58 einfließen kann.

Des weiteren wird bei der zuerst beschriebenen Vorgehensweise, bei der die Bohrung 58 mittels eines Stopfens verschlossen wird, die Bohrung 58 vorzugsweise mit einem kleineren Durchmesser ausgebildet als die Bohrung 60, wodurch das Einsetzen des Stopfens in die Bohrung 58 erleichtert wird.

Nach dem Aushärten des vollständig eingefüllten Klebstoffs 46 in das Verbindungsteil 44 wird schließlich die Bohrung 56 und die Bohrung 54 durch den Klebstoff 46 in den Spalt 64 hindurch eingebracht, so daß die Bohrung 54 und die Bohrung 56 automatisch gegen das Verbindungsteil 44 abgedichtet sind. Der Klebstoff 46 dichtet den ersten Kanal 18 von dem zweiten Kanal 22 im proximalen Bereich vollständig ab, so daß eine Vermischung von Spülflüssigkeit und Saugflüssigkeit durch den Klebstoff 46 sicher vermieden wird.

Im abschließenden Schritt wird dann der Deckel 48 an dem Verbindungsteil 44 befestigt. Das Verbindungsteil 44 und der Schaft 12 einschließlich des Deckels 48 stellen dann eine fest miteinander verbundene einteilige Einheit dar.

## Patentansprüche

1. Medizinisches Instrument zum Saugen und Spülen, mit einem langerstreckten Schaft (12), der einen ersten Kanal (18), der als Saugkanal dient, und einen zweiten Kanal (22) aufweist, der als Spülkanal dient, wobei der Schaft (12) ein im Querschnitt geschlossenes erstes Rohr (26) aufweist, in dem der eine der Kanäle (18, 22) vorhanden ist, wobei der Schaft (12) ein über seine Länge im Querschnitt offenes zweites Rohr (28) aufweist, das mit seiner offenen Längsseite auf eine Außenseite (34) des ersten Rohrs (26) aufgesetzt und an diesem dichtend befestigt ist, wobei der andere Kanal im Zwischenraum zwischen der Außenseite (34) des ersten Rohrs (26) und einer Innenseite (36) des zweiten Rohrs (28) ausgebildet ist, wobei ferner am proximalen Ende (16) des Schafts (12) ein den Schaft (12) umgebendes Verbindungsteil (44) zum Verbinden des Schafts (12) mit einem Handgriff angeordnet ist, **dadurch gekennzeichnet, daß** der Schaft (12) in das Verbindungsteil (44) mittels eines aushärtenden Klebstoffes (46) eingeklebt ist und daß der Klebstoff (46) das erste Rohr (26) und das zweite Rohr (28) in deren proximalem Endbereich gegeneinander abdichtet, wobei ein proximales offenes Ende (68) des zweiten Rohrs (28) mittels desselben Klebstoffs (46) oder eines weiteren Klebstoffs verschlossen ist.

2. Instrument nach Anspruch 1, **dadurch gekennzeichnet, daß** der Zwischenraum zwischen der Außenseite (34) des ersten Rohrs (26) und der Innenseite (36) des zweiten Rohrs (28) sichelförmig mit geringer Scheitelhöhe ausgebildet ist.

3. Instrument nach Anspruch 1 oder 2, **dadurch gekennzeichnet, daß** das erste Rohr (26) weiterhin einen dritten Kanal (38) zur Aufnahme eines Optikschafts eines Endoskops aufweist, und daß das zweite Rohr (28) auf derjenigen Außenseite (34) des ersten Rohrs (26) aufgesetzt ist, die von dem verbleibenden freien Querschnitt in dem ersten Rohr (26), der den ersten oder zweiten Kanal (18, 22) bildet, abgewandt ist.

4. Instrument nach einem der Ansprüche 1 bis 3, **dadurch gekennzeichnet, daß** das zweite Rohr (28) die Außenseite (34) des ersten Rohrs, an der es aufgesetzt ist, geringfügig in Längsrichtung überragt.

5. Instrument nach einem der Ansprüche 1 bis 4, **dadurch gekennzeichnet, daß** ein distales Ende des zweiten Rohrs (28) zum ersten Rohr (26) hin umgebördelt ist.

6. Instrument nach einem der Ansprüche 1 bis 5, **dadurch gekennzeichnet, daß** ein distales Ende des ersten Rohrs (26) auf der dem zweiten Rohr (28) abgewandten Außenseite zum zweiten Rohr (28) hin umgebördelt ist.

7. Instrument nach einem der Ansprüche 1 bis 6, **dadurch gekennzeichnet, daß** der Saugkanal im ersten Rohr (26) und der Spülkanal im Zwischenraum zwischen der Außenseite (34) des ersten Rohrs (26) und der Innenseite (36) des zweiten Rohrs (28) angeordnet ist.

8. Instrument nach einem der Ansprüche 1 bis 7, **dadurch gekennzeichnet, daß** das zweite Rohr (28) entlang seiner Längsränder (30, 32) mit der Außenseite (34) des ersten Rohrs (26) durch Kleben, Löten oder Schweißen fest verbunden ist.

9. Instrument nach Anspruch 8, **dadurch gekennzeichnet, daß** das zweite Rohr (28) entlang seiner Längsränder (30, 32) mit der Außenseite des erste Rohrs durch Laserschweißen verbunden ist.

10. Verfahren zum Herstellen eines medizinischen Instruments zum Saugen und Spülen, das einen langerstreckten Schaft (12) aufweist, der einen ersten Kanal (18), der als Saugkanal dient, und einen zweiten Kanal (22) aufweist, der als Spülkanal dient, wobei der Schaft (12) ein erstes Rohr (26) aufweist, in dem der eine der Kanäle (18, 22) angeordnet ist, wobei ein über seine Länge im Querschnitt offenes zweites Rohr (28) mit seiner offenen Längsseite auf eine Außenseite (34) des ersten Rohrs (26) aufgesetzt und an diesem dichtend befestigt wird, derart, daß zwischen der Außenseite (34) des ersten Rohrs (26) und einer Innenseite (36) des zweiten Rohrs (28) ein in Längsrichtung durchgehender Zwischenraum verbleibt, **dadurch gekennzeichnet, daß** die miteinander verbundene Einheit aus erstem Rohr (26) und zweitem Rohr (28) anschließend mit einem Verbindungsteil (44) zum Verbinden des Schafts (12) mit einem Handgriff verbunden wird, wobei in dem Verbindungsteil (44) eine Längsbohrung (62) vorhanden ist, oder in das Verbindungsteil (44) vor dem Verbinden mit dem Schaft (12) eine Längsbohrung (62) eingebracht wird, in die der Schaft (12) eingeführt und anschließend mittels eines Klebstoffes (46) eingeklebt wird, so daß der Klebstoff (46) das erste Rohr (26) und das zweite Rohr (28) in deren proximalen Endbereich gegeneinander abdichtet, wobei ein proximales offenes Ende (68) des zweiten Rohrs (28) mittels desselben Klebstoffes (46) oder eines weiteren Klebstoffes verschlossen wird.

11. Verfahren nach Anspruch 10, **dadurch gekennzeichnet, daß** das zweite Rohr (28) mit dem ersten Rohr (26) durch Kleben, Löten oder Schweißen fest verbunden wird.

12. Verfahren nach Anspruch 11, **dadurch gekennzeichnet, daß** das zweite Rohr (28) mit dem ersten Rohr (26) durch Laserschweißen verbunden wird.

13. Verfahren nach einem der Ansprüche 10 bis 12, **dadurch gekennzeichnet, daß** vor dem Verbinden des zweiten Rohrs (28) mit dem ersten Rohr (26) in das zweite Rohr (28) eine seitliche Bohrung (58) eingebracht wird.

14. Verfahren nach Anspruch 13, **dadurch gekennzeichnet, daß** die seitliche Bohrung (58) in dem zweiten Rohr (28) vor dem Einbringen des Klebstoffes (46) mittels eines anschließend wieder abnehmbaren Stopfens verschlossen wird.

15. Verfahren nach einem der Ansprüche 10 bis 14, **dadurch gekennzeichnet, daß** nach dem Einkleben des Schafts (12) in das Verbindungsteil (44) in das erste Rohr (26) an einer Stelle, die nicht von dem zweiten Rohr (28) bedeckt ist, eine seitliche Bohrung (54) durch den ausgehärteten Klebstoff (46) hindurch eingebracht wird.

## Claims

1. A medical instrument for suction and irrigation, comprising an elongate shaft (12) with a first channel (18) serving as suction channel and with a second channel (22) serving as irrigation channel, the shaft (12) having a first tube (26) which is closed in cross section and in which one of the channels (18, 22) is present, wherein the shaft (12) has a second tube (28) which is open in cross section along its length and is placed with its open lengthwise side on an outside face (34) of the first tube (26) and secured sealingly thereon, the other channel is formed in the space between the outside face (34) of the first tube (26) and an inside face (36) of the second tube (28), wherein a connection part (44) surrounding the shaft (12) and used to connect said shaft (12) to a handgrip is arranged at the proximal end (16) of the shaft (12), **characterized in that** said shaft (12) is bonded into the connection part (44) by means of a curing adhesive (46), and that the adhesive (46) seals off the first tube (26) and the second tube (28) from one another in their proximal end area, wherein a proximal open end (68) of the second tube (28) is closed by means of the same adhesive (46) or by means of another adhesive.

2. The instrument of claim 1, **characterized in that** the space between the outside face (34) of the first tube (26) and the inside face (36) of the second tube (28) is designed in the shape of a crescent with a low height.

3. The instrument of claim 1 or 2, **characterized in that** the first tube (26) also has a third channel (38) for receiving an optical shaft of an endoscope, and wherein the second tube (28) is placed on that outside face (34) of the first tube (26) directed away from the remaining free cross section in the first tube (26) which forms the first or second channel (18, 22).

4. The instrument of any one of claims 1 through 3, **characterized in that** the second tube (28) protrudes slightly in the longitudinal direction beyond the outside face (34) of the first tube on which it is placed.

5. The instrument of any one of claims 1 through 4, **characterized in that** a distal end of the second tube (28) is flanged in toward the first tube (26).

6. The instrument of any one of claims 1 through 5, **characterized in that** a distal end of the first tube (26), on the outside face directed away from the second tube (28), is flanged in toward said second tube (28).

7. The instrument of any one of claims 1 through 6, **characterized in that** the suction channel is arranged in the first tube (26) and the irrigation channel is arranged in the space between the outside face (34) of the first tube (26) and the inside face (36) of the second tube (28).

8. The instrument of any one of claims 1 through 7, **characterized in that** the second tube (28) along its lengthwise edges (30, 32) is fixedly connected to the outside face (34) of the first tube (26) by adhesive bonding, soldering or welding.

9. The instrument of claim 8, **characterized in that** the second tube (28) along its lengthwise edges (30, 32) is connected to the outside face of the first tube by laser welding.

10. A method for producing a medical instrument for suction and irrigation, which instrument comprises an elongate shaft (12) with a first channel (18) serving as suction channel and with a second channel (22) serving as irrigation channel, the shaft (12) having a first tube (26) in which one of the channels (18, 22) is arranged, wherein a second tube (28) which is open in cross section along its length is placed with its open lengthwise side on an outside face (34) of the first tube (26) and is secured sealingly thereon in such a way that a space continuous in the longitudinal direction remains between the outside face (34) of the first tube (26) and an inside face (36) of the second tube (28), **characterized in that** the interconnected unit comprising the first tube (26) and the second tube (28) is subsequently connected to a connection part (44) for connecting the shaft (12) to a handgrip, wherein a longitudinal bore (62) is present in the connection part (44), or a longitudinal bore (62) is formed in the connection part (44) before connection to the shaft (12), into which bore (62) the shaft (12) is introduced and then bonded by means of an adhesive (46) so that the adhesive (46) seals off the first tube (26) and the second tube (28) from one another in their proximal end area, wherein a proximal open end (68) of the second tube (28) is closed by means of the same adhesive (46) or by means of another adhesive.

11. The method of claim 10, **characterized in that** the second tube (28) is fixedly connected to the first tube (26) by adhesive bonding, soldering or welding.

12. The method of claim 11, **characterized in that** the second tube (28) is connected to the first tube (26) by laser welding.

13. The method of any one of claims 10 through 12, **characterized in that**, before the second tube (28) is connected to the first tube (26), a lateral bore (58) is formed in the second tube (28).

14. The method of claim 13, **characterized in that**, before introduction of the adhesive (46), the lateral bore (58) in the second tube (28) is closed off by means of a subsequently removable stopper.

15. The method of claim anyone of claims 10 through 14, **characterized in that**, after the shaft (12) has been bonded adhesively into the connection part (44), a lateral bore (54) is formed right through the cured adhesive (46) into the first tube (26) at a location not covered by the second tube (28).

## Revendications

1. Instrument médical pour l'aspiration et l'irrigation, comportant une tige (12) allongée, qui est munie d'un premier conduit (18), qui fait fonction de conduit d'aspiration, et d'un deuxième conduit (22) qui fait fonction de conduit d'irrigation, la tige (12) comportant un premier tube (26) à section fermée, dans lequel est disposé l'un des conduits (18, 22), la tige (12) comportant un deuxième tube (28) à section ouverte sur toute sa longueur, lequel est posé avec son côté longitudinal ouvert sur une face extérieure (34) du premier tube (26) et est fixé de manière étanche à celui-ci, l'autre conduit étant réalisé dans l'espace intermédiaire entre la face extérieure (34) du premier tube (26) et une face intérieure (36) du deuxième tube (28), sachant qu'en outre sur l'extrémité proximale (16) de la tige (12) est agencé un élément d'assemblage (44), qui entoure la tige (12) et est destiné à assembler la tige (12) à une poignée, **caractérisé en ce que** la tige (12) est collée dans l'élément d'assemblage (44) au moyen d'une colle (46) durcissable, et **en ce que** la colle (46) assure l'étanchéité du premier tube (26) par rapport au deuxième tube (28) dans leur zone d'extrémité proximale, sachant qu'une extrémité proximale (68) ouverte du deuxième tube (28) est obturée au moyen de la même colle (46) ou d'une autre colle.

2. Instrument selon la revendication 1, **caractérisé en ce que** l'espace intermédiaire entre la face extérieure (34) du premier tube (26) et la face intérieure (36) du deuxième tube (28) est réalisé en forme de croissant avec une faible hauteur au sommet.

3. Instrument selon la revendication 1 ou 2, **caractérisé en ce que** le premier tube (26) comporte, en outre, un troisième conduit (38) destiné à recevoir une tige optique d'un endoscope, et **en ce que** le deuxième tube (28) est posé sur la face extérieure (34) du premier tube (26), laquelle est opposée à la section libre subsistante dans le premier tube (26) qui forme le premier ou le deuxième conduit (18, 22).

4. Instrument selon l'une quelconque des revendications 1 à 3, **caractérisé en ce que** le deuxième tube (28) s'avance quelque peu dans la direction longitudinale au-delà de la face extérieure (34) du premier tube sur lequel il est posé.

5. Instrument selon l'une quelconque des revendications 1 à 4, **caractérisé en ce qu'**une extrémité distale du deuxième tube (28) est rabattue vers le premier tube (26).

6. Instrument selon l'une quelconque des revendications 1 à 5, **caractérisé en ce qu'**une extrémité distale du premier tube (26) est rabattue vers le deuxième tube (28), sur la face extérieure opposée au deuxième tube (28).

7. Instrument selon l'une quelconque des revendications 1 à 6, **caractérisé en ce que** le conduit d'aspiration est agencé dans le premier tube (26) et le conduit d'irrigation est agencé dans l'espace intermédiaire entre la face extérieure (34) du premier tube (26) et la face intérieure (36) du deuxième tube (28).

8. Instrument selon l'une quelconque des revendications 1 à 7, **caractérisé en ce que** le deuxième tube (28) est assemblé de manière fixe le long de ses bords longitudinaux (30, 32) par collage, brasage ou soudage avec la face extérieure (34) du premier tube (26).

9. Instrument selon la revendication 8, **caractérisé en ce que** le deuxième tube (28) est assemblé le long de ses bords longitudinaux (30, 32) par soudage laser avec la face extérieure du premier tube.

10. Procédé de réalisation d'un instrument médical pour l'aspiration et l'irrigation, qui comporte une tige (12) allongée, qui est munie d'un premier conduit (18), qui fait fonction de conduit d'aspiration, et d'un deuxième conduit (22) qui fait fonction de conduit d'irrigation, la tige (12) comportant un premier tube (26), dans lequel est disposé l'un des conduits (18, 22), sachant qu'un deuxième tube (28) à section ouverte sur toute sa longueur est posé avec son côté longitudinal ouvert sur une face extérieure (34) du premier tube (26) et est fixé de manière étanche à celui-ci, de telle sorte qu'un espace intermédiaire, continu dans la direction longitudinale, subsiste entre la face extérieure (34) du premier tube (26) et une face intérieure (36) du deuxième tube (28), **caractérisé en ce que** l'unité formée par l'assemblage de l'un à l'autre du premier tube (26) et du deuxième tube (28), est assemblée ensuite à un élément d'assemblage (44) destiné à assembler la tige (12) à une poignée, sachant que dans l'élément d'assemblage (44) est prévue une forure longitudinale (62) ou dans l'élément d'assemblage (44) a été ménagée, avant l'assemblage avec la tige (12), une forure longitudinale (62), dans laquelle la tige (12) est introduite et est collée ensuite au moyen d'une colle (46), de telle sorte que la colle (46) assure l'étanchéité du premier tube (26) par rapport au deuxième tube (28) dans leur zone d'extrémité proximale, sachant qu'une extrémité proximale (68) ouverte du deuxième tube (28) est obturée au moyen de la même colle (46) ou d'une autre colle.

11. Procédé selon la revendication 10, **caractérisé en ce que** le deuxième tube (28) est assemblé de manière fixe au premier tube (26) par collage, brasage ou soudage.

12. Procédé selon la revendication 11, **caractérisé en ce que** le deuxième tube (28) est assemblé par soudage laser au premier tube (26).

13. Procédé selon l'une quelconque des revendications 10 à 12, **caractérisé en ce qu'**une forure latérale (58) est ménagée dans le deuxième tube (28) avant l'assemblage du deuxième tube (28) avec le premier tube (26).

14. Procédé selon la revendication 13, **caractérisé en ce que** la forure latérale (58) dans le deuxième tube (28) est obturée, avant l'introduction de la colle (46), au moyen d'un bouchon apte à être retiré ultérieurement.

15. Procédé selon l'une quelconque des revendications 10 à 14, **caractérisé en ce qu'**après le collage de la tige (12) dans l'élément d'assemblage (44), une forure latérale (54) est ménagée, à travers la colle (46) durcie, dans le premier tube (26) à un emplacement qui n'est pas couvert par le deuxième tube (28).
